(19) Europäisches Patentamt
European Patent Office
Office européen des brevets

(11) **EP 4 047 102 A2**

(12) **EUROPEAN PATENT APPLICATION**
published in accordance with Art. 153(4) EPC

(43) Date of publication:
**24.08.2022 Bulletin 2022/34**

(51) International Patent Classification (IPC):
***C12Q 1/6886*** (2018.01)

(21) Application number: **20877421.6**

(22) Date of filing: **08.10.2020**

(86) International application number:
**PCT/KR2020/013801**

(87) International publication number:
**WO 2021/075797 (22.04.2021 Gazette 2021/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.10.2019 KR 20190127218**

(71) Applicant: **Gencurix Inc.**
**Seoul 08394 (KR)**

(72) Inventors:
• **CHO, Sang Rae**
  **Seoul 08394 (KR)**
• **MOON, Young Ho**
  **Seoul 08394 (KR)**
• **HAN, Jin Il**
  **Seoul 08394 (KR)**

(74) Representative: **Hoefer & Partner Patentanwälte mbB**
**Pilgersheimer Straße 20**
**81543 München (DE)**

(54) **COMPOSITION FOR DIAGNOSING LIVER CANCER BY USING CPG METHYLATION CHANGES IN SPECIFIC GENES, AND USE THEREOF**

(57) The present invention relates to a composition, a kit, a nucleic acid chip and a method which allow the diagnosis of liver cancer by detecting methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2. The present invention enables accurate and rapid diagnosis of liver cancer, and also enables early diagnosis.

FIG. 14

**EP 4 047 102 A2**

**Description**

**TECHNICAL FIELD**

The present invention relates to a composition, a kit, a nucleic acid chip and a method which allow the diagnosis of liver cancer by detecting methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.

**BACKGROUND ART**

This application claims the priority of Korean Patent Application No. 2019-0127218, filed on October 14, 2019, the entirety of which is a reference of the present application.

Liver cancer is one of cancers with high incidence worldwide. In Korea, the mortality from liver cancer is very high at 23 per 100,000 people, and about 10% of the total mortality in Koreans is related to hepatitis, cirrhosis and liver cancer. Liver cancer is difficult to be diagnosed early because there are no perceptual symptoms. In general, since liver cancer is usually detected in a stage of advanced carcinoma that cannot be treated properly, the treatment is very limited and the prognosis is also very poor. Since the prognosis of liver cancer varies greatly depending on the cancer progression at the time of diagnosis, early detection of liver cancer patients is very important to increase the survival rate of liver cancer patients.

On the other hand, epigenetics is a field that studies the regulation of gene expression in a state in which the base sequence of DNA is not changed. The epigenetics is to study the regulation of gene expression through epigenetic variations, such as DNA methylation, acetylation, methylation, phosphorylation, and ubiquitination of miRNAs or histones, and the like.

Among them, the DNA methylation is the most studied epigenetic variation. The epigenetic variation may result in gene function variations and changes to tumor cells. Accordingly, the DNA methylation is associated with the expression (or inhibition and induction) of disease-regulatory genes in cells, and recently, cancer diagnosis methods through measurement of DNA methylation have been proposed. In particular, since cancer-specific methylation occurs in advance even in precancerous tissues, the detection of cancer-specific methylation is highly likely to be used for diagnosis of cancer.

Therefore, it is necessary to develop effective liver cancer-specific methylation markers capable of predicting the risk of liver cancer.

**DISCLOSURE**

**TECHNICAL PROBLEM**

Therefore, the present inventors found that CpG sites of specific genes were hypermethylated state in liver cancer, developed a composition, a kit, a nucleic acid chip, and a method capable of diagnosing liver cancer by detecting the methylation levels, and then completed the present invention.

An object of the present invention is to provide a composition for diagnosing liver cancer comprising a preparation for measuring methylation levels of CpG sites of specific genes.

Another object of the present invention is to provide a kit for diagnosing liver cancer comprising a PRC primer pair for amplifying fragments including CpG sites of specific genes and a sequencing primer for pyrosequencing a PCR product amplified by the primer pair.

Yet another object of the present invention is to provide a nucleic acid chip for diagnosing liver cancer in which probes capable of hybridizing with fragments including CpG sites of specific genes under a stringent condition are immobilized.

Still another object of the present invention is to provide a method for diagnosing liver cancer comprising measuring and comparing methylation levels of CpG sites of specific genes from different samples.

Still yet another object of the present invention is to provide a use of a preparation for measuring methylation levels of CpG sites of genes for preparing a preparation for diagnosing liver cancer.

**TECHNICAL SOLUTION**

In order to achieve the object, there is provided a composition for diagnosing liver cancer comprising a preparation for measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.

In order to achieve another object, there is provided a kit for diagnosing liver cancer comprising a primer pair for amplifying fragments including CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.

In order to achieve yet another object, there is provided a nucleic acid chip for diagnosing liver cancer immobilized with probes capable of hybridizing with fragments including CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.

In order to achieve still another object, there is provided a method for diagnosing liver cancer comprising measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 from samples of patients suspected of having liver cancer; and

comparing the measured methylation levels with methylation levels of CpG sites of the same genes in a normal control sample.

In order to achieve still yet another object, there is provided a use of a preparation for measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 for preparing a preparation for diagnosing liver cancer.

Unless otherwise defined, all technical and scientific terms used herein have the same meaning as commonly understood by those skilled in the art. The following references provide one skill with general definitions of various terms used in the present specification: Singleton et al., DICTIONARY OF MICROBIOLOGY AND MOLECULAR BIOLOTY(2th ed. 1994); THE CAMBRIDGE DICTIONARY OF SCIENCE AND TECHNOLOGY(Walkered., 1988); and Hale & Marham, THE HARPER COLLINS DICTIONARY OF BIOLOGY.

Hereinafter, the present invention will be described in detail.

**The present invention relates to a composition for diagnosing liver cancer comprising a preparation for measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.**

As used herein, the term "methylation" means that a methyl group is attached to a base constituting DNA. Preferably, in the present invention, the methylation refers to whether methylation occurs in cytosine of a specific CpG site of a specific gene. When the methylation occurs, the binding of a transcription factor is disturbed to inhibit the expression of the specific gene. Conversely, when unmethylation or hypomethylation occurs, the expression of the specific gene is increased.

In genomic DNA of mammalian cells, in addition to A, C, G, and T, there is the fifth base called 5-methylcytosine (5-mC) with a methyl group attached to the fifth carbon of a cytosine ring. The methylation of 5-methylcytosine occurs only at C of CG dinucleotide (5'-mCG-3') called CpG, and methylation of CpG inhibits the expression of alu or transposon and a repeating sequence of a genome. In addition, since 5-mC of the CpG is naturally deaminated to be easily converted to thymine (T), the CpG is a site where most epigenetic changes frequently occur in mammalian cells.

As used herein, the term "measurement of the methylation level" refers to measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2, and the methylation levels may be measured by a detection method according to the treatment of bisulfite or a bisulfite-free detection method. The methylation level may be measured by methylation-specific PCR, for example, methylation-specific polymerase chain reaction (MSP), real time methylation-specific polymerase chain reaction (PCR), PCR using a methylated DNA-specific binding protein, or quantitative PCR. Alternatively, the methylation level may be measured by automatic sequencing such as pyrosequencing and bisulfite sequencing, but is not limited thereto. In addition, the methylation level may be measured using a detection method using a ten-eleven translocation protein (TET protein) as the bisulfite-free detection method (see Nature Biotechnology, volume 37, pages 424-429 (2019)). The TET protein is an enzyme that acts on DNA and is involved in chemical changes of bases, and when bisulfite is treated, all Cs except for methylated C are converted to T bases, whereas in the Tet protein, only the methylated C is converted to T to enable effective detection.

Preferably, the CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 refer to CpG sites on DNAs of the genes. The DNA of the gene is a concept including all of a series of structural units required for expression of the gene and operably linked to each other, and may include, for example, a promoter region, an open reading frame (ORF) and a terminator region. Accordingly, the CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 exist in the promoter regions, the open reading frames (ORFs) or the terminator regions of the corresponding genes.

Preferably, in the present invention, the measuring of the methylation levels of the CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 may mean measuring methylation levels of cytosines in CpG sites of genes shown in Table 1 below.

[Table 1]

| Symbol | Genome Build | Chromosome | Region |
|---|---|---|---|
| FAM110A | GRCh37 | 20 | 825267-826276 |
| FAR1 | GRCh37 | 11 | 13689588-13690724 |
| VIM | GRCh37 | 10 | 17270430-17272617 |
| LDHB | GRCh37 | 12 | 21810488-21810766 |
| LIPE | GRCh37 | 19 | 42901016-42901375 |
| INAFM1 | GRCh37 | 19 | 47776370-47778740 |
| ATL1 | GRCh37 | 14 | 51026872-51027570 |
| CELF6 | GRCh37 | 15 | 72611946-72612802 |
| MTHFD2 | GRCh37 | 2 | 74425444-74426423 |
| PAK1 | GRCh37 | 11 | 77122736-77123088 |
| NXPE3 | GRCh37 | 3 | 101497830-101498648 |
| SLC25A36 | GRCh37 | 3 | 140660334-140661602 |
| VANGL2 | GRCh37 | 1 | 160370112-160370658 |

In the present invention, it is characterized that the CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 are located between +/- 2000 bases (2 kb) from transcription start sites (TSS) of the genes.

In the present invention, a base sequence of a human genome chromosomal region was expressed according to The February 2009 Human reference sequence (GRCh37), but the expression of a specific sequence of the human genome chromosomal region may be slightly changed as the study results on the genomic sequence are updated, and the expression of the human genome chromosomal region of the present invention may vary according to the change. Accordingly, in the human genome chromosomal region expressed according to The February 2009 Human reference sequence (GRCh 37) of the present invention, since a human reference sequence has been updated after the filing date of the present invention, even if the expression of the human genome chromosomal region is changed differently from now, it will be apparent that the scope of the present invention affects the changed human genome chromosomal region. These changes may be easily seen by those with ordinary skill in the art to which the present invention pertains.

In the present invention, the preparation for measuring the methylation level of the CpG site may include a compound of modifying a cytosine base or a methylation-sensitive restriction enzyme, primers specific to methylated allele sequences of any one or more genes selected from the group consisting of FAM110A, FAR1, VIM, LDHB, LIPE, INAFM1, ATL1, CELF6, MTHFD2, PAK1, NXPE3, SLC25A36 and VANGL2, and primers specific to unmethylated allele sequences.

The compound of modifying the cytosine base is a compound of modifying unmethylated cytosine or methylated cytosine, and may be bisulfite or a salt thereof, preferably sodium bisulfite of modifying the unmethylated cytosine, or a TET protein of modifying the methylated cytosine, but is not limited thereto. Methods for detecting whether the CpG site is methylated by modifying the cytosine base are well known in the art (WO01/26536; US2003/0148326A1).

In addition, the methylation-sensitive restriction enzyme may be a restriction enzyme capable of specifically detecting methylation of the CpG site, and may be a restriction enzyme containing CG as a recognition site of the restriction enzyme. Examples thereof include SmaI, SacII, EagI, HpaII, MspI, BssHII, BstUI, NotI, and the like, but are not limited thereto. Depending on methylation or unmethylation at C of the restriction enzyme recognition site, cleavage by the restriction enzyme varies and may be detected through PCR or southern blot analysis. Methylation-sensitive restriction enzymes other than the restriction enzymes are well known in the art.

The primers may include primers specific to methylated allele sequences of any one or more genes selected from the group consisting of FAM110A, FAR1, VIM, LDHB, LIPE, INAFM1, ATL1, CELF6, MTHFD2, PAK1, NXPE3, SLC25A36 and VANGL2, and primers specific to unmethylated allele sequences.

In the present invention, the term "primer" refers to a nucleic acid sequence having a short free 3-terminal hydroxyl group, and a short nucleic acid sequence capable of forming a base pair with a complementary template and serving as a starting point for copying a template strand. The primer may initiate DNA synthesis in the presence of a reagent for polymerization (i.e., DNA polymerase or reverse transcriptase) and four different nucleoside triphosphates in appropriate buffer and temperature. In addition, the primer is a sense and antisense nucleic acid with a sequence of 7 to 50 nucleotides and may incorporate an additional feature without changing the basic properties of the primer that serves as an initiation site of DNA synthesis.

The primer of the present invention may be preferably designed according to a sequence of a specific CpG site for analyzing methylation, and more preferably, may be at least one selected the group consisting of a primer pair capable of specifically amplifying cytosine that is methylated to be unmodified by bisulfite, a primer pair capable of specifically amplifying cytosine that is not methylated to be modified by bisulfite, and a primer pair capable of specifically amplifying cytosine that is methylated to be modified by a Tet-based protein, and a primer pair capable of specifically amplifying cytosine that is not methylated and has not been modified by a Tet-based protein.

**Accordingly, the present invention provides a kit for diagnosing liver cancer comprising a primer pair for amplifying fragments including CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.**

In the composition and the kit, in addition to the preparation, polymerase agarose, a buffer solution required for electrophoresis, and the like may be additionally included.

**Further, the present invention provides a nucleic acid chip for diagnosing liver cancer immobilized with probes capable of hybridizing with fragments including CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.**

In the present invention, the term "nucleic acid" refers to oligonucleotides, nucleotides, polynucleotides or fragments thereof, single- or double-stranded genomic or synthetic-origin DNA or RNA, sense or antisense-stranded genomic or synthetic-origin DNA or RNA, and peptide nucleic acid (PNA) or natural or synthetic-origin DNA or RNA analog material. If the nucleic acid is RNA, it will be apparent to those skilled in the art that instead of deoxynucleotides A, G, C and T, ribonucleotides A, G, C and U are substituted, respectively.

Since the methylation starts from the outside of a regulatory site of a gene and proceeds to the inside thereof, the gene involved in cell transformation may be diagnosed early by detecting the methylation outside the regulatory site.

Accordingly, it is possible to early diagnose cells likely to form liver cancer using the methylated gene marker. When a gene confirmed to be methylated in cancer cells is methylated in cells appearing to be clinically or morphologically normal, the normal-appearing cells are being cancerizated. Therefore, it is possible to diagnose liver cancer early by confirming the methylation of a liver cancer-specific gene in normal-appearing cells.

**Further, the present invention provides a method for diagnosing liver cancer comprising measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 from samples of patients suspected of having liver cancer; and comparing the measured methylation levels with methylation levels of CpG sites of the same genes in a normal control sample.**

The method for measuring the methylation level may be selected from the group consisting of PCR, methylation specific PCR, real time methylation specific PCR, PCR using a methylated DNA-specific binding protein, measuring whether there is methylation using a methylation-sensitive restriction enzyme, quantitative PCR, DNA chip, pyrosequencing and bisulfite sequencing, but is not limited thereto.

Specifically, the method of methylation-specific PCR is a method of designing and using different types of primers depending on whether CpG dinucleotide is methylated as a primer to perform PCR after treating a sample DNA with bisulfite. If the primer binding site has been methylated, PCR proceeds with the methylated primer, and if not methylated, PCR proceeds with a normal primer. That is, the method is a method of treating the sample DNA with bisulfite, performing PCR using two types of primers at the same time, and comparing the results.

The real time methylation specific PCR is to convert the methylation-specific PCR method to a real-time measurement method and to perform real time PCR by treating genomic DNA with bisulfite, designing PCR primers corresponding to methylation, and using these primers. At this time, there are two methods: a detection method using a TanMan probe complementary to an amplified base sequence and a detection method using Sybergreen. Accordingly, the real time methylation specific PCR may selectively quantitative-analyze only methylated DNA. In this case, the method is a method of preparing a standard curve using an in vitro methylated DNA sample, and quantitatively analyzing the methylation level by amplifying a gene without a 5'-CpG-3' sequence in a base sequence together as a negative control for standization.

In the method for measuring the methylation using the methylation-sensitive restriction enzyme, the methylation-sensitive restriction enzyme uses CpG dinucleotide as an action site, and dose not act as the enzyme when this site is methylated. Therefore, when the sample DNA is treated with the methylation-sensitive restriction enzyme and then amplified by PCR to include an enzyme target site, the restriction enzyme does not act in the case of methylation, but is amplified by PCR, but the unmethylated normal site is cleaved by the restriction enzyme and not amplified by PCR, thereby measuring whether a specific DNA site is methylated.

In the PCR or DNA chip method using the methylated DNA-specific binding protein, when a protein that specifically binds only to methylated DNA is mixed with DNA, the protein specifically binds only to the methylated DNA, so that only the methylated DNA may be selectively isolated. After mixing genomic DNA with the methylated DNA-specific binding protein, only the methylated DNA is selectively isolated. This is a method of amplifying these isolated DNAs using a PCR primer corresponding to an intron site, and then measuring whether methylation occurs by agarose electrophoresis. In addition, the methylation may be measured by quantitative PCR, and the methylated DNA isolated by the methylated DNA-specific binding protein is labeled with a fluorescent dye and hybridized to a DNA chip integrated with a complementary probe to measure the methylation. Here, the methylated DNA-specific binding protein is not limited to MBD2bt.

In addition, pyrosequencing of bisulfite-treated DNA is based on the following principle. When methylation occurs at the CpG dinucleotide site, 5-methylcytosine (5-mC) is formed, wherein the modified base is converted to uracil upon treatment with bisulfite. If the CpG dinucleotide has been methylated when the DNA extracted from the sample is treated with bisulfite, the CpG dinucleotide is preserved as cytosine and the remaining unmethylated cytosines are converted to uracils. Sequencing of the bisulfite-treated DNA may be preferably performed using a pyrosequencing method. The detailed description of pyrosequencing is known in the prior arts [Ronaghi et al, Science 1998 Jul 17, 281(5375), 363-365; Ronaghi et al, Analytical Biochemistry 1996 Nov 1, 242(1), 84-9; Ronaghi et al. Analytical Biochemistry 2000 Nov 15, 286 (2): 282-288; Nyr, P. Methods Mol Biology 2007, 373, 114].

On the other hand, by the bisulfite-free detection method using the Tet protein, only methylated C may be converted to T using the Tet protein to detect the base at the methylated site (see LIU, Yibin, et al., Nature Biotechnology volume 37, pages 424). - 429 (2019)).

When the methylation occurs at the CpG dinucleotide site so that cytosine is formed as 5-methylcytosine (5-mC), the CpG dinucleotide has been methylated when treated with the ten-eleven translocation (Tet) protein to be converted to uracil, and unmethylated cytosine is preserved. The sequencing of Tet-treated DNA is not limited only to the pyrosequencing method, but may be performed by using methods such as methylation-sensitive PCR (MSP), microarray, and next generation sequencing (NGS).

Preferably, the method for diagnosing liver cancer of the present invention may be performed by a method characterized by including steps of a) obtaining a sample from a subject, b) obtaining genomic DNA from the sample, c) treating the obtained genomic DNA with a compound of modifying an unmethylated cytosine base, d) obtaining a PCR product by amplifying the treated DNA by PCR using a primer capable of amplifying any one or more CpG sites selected from the group consisting of FAM110A, FAR1, VIM, LDHB, LIPE, INAFM1, ATL1, CELF6, MTHFD2, PAK1, NXPE3, SLC25A36 and VANGL2, and e) measuring the methylation level of the PCR product.

The obtaining of the genomic DNA in step b) may be performed using a phenol/chloroform extraction method commonly used in the art, an SDS extraction method (Tai et al., Plant Mol. Biol. Reporter, 8: 297-303, 1990), a CTAB separation method (Cetyl Trimethyl Ammonium Bromide; Murray et al., Nuc) Res., 4321-4325, 1980) or a commercially available DNA extraction kit.

In the present invention, the term "sample" refers to a wide range of bodily fluids, including all biological fluids obtained from individuals, bodily fluids, cell lines, tissue culture, etc., depending on a type of analysis to be performed. Methods for obtaining bodily fluids and tissue biopsies from mammals are generally well known, and in the present invention, the samples may be preferably selected from the group consisting of human derivatives including tissues, cells, blood, plasma, serum, feces, and urine. Since abnormal methylation changes in cancer tissues show significant similarities to methylation changes in genomic DNA obtained from biological samples such as cells, whole blood, serum, plasma, saliva, sputum, cerebrospinal fluid, or urine, in the case of using the marker of the present invention, with respect to the prediction of the occurrence of liver cancer, there is an advantage that it is possible to be easily diagnosed through blood, bodily fluids, or the like.

**Further, the present invention provides a use of a preparation for measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 for preparing a preparation for diagnosing liver cancer.**

ADVANTAGEOUS EFFECTS

As described above, since hypermethylation of the CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 occurs specifically in liver cancer, it is possible to accurately and rapidly liver cancer and early diagnose the liver cancer by using the composition, the kit, the chip or the method according to the present invention.

## DESCRIPTION OF DRAWINGS

FIG. 1 is a result of confirming methylation information of a FAM110A gene in a total of 33 cancer types.

FIG. 2 is a result of confirming methylation information of a FAR1 gene in a total of 33 cancer types.

FIG. 3 is a result of confirming methylation information of a VIM gene in a total of 33 cancer types.

FIG. 4 is a result of confirming methylation information of a LDHB gene in a total of 33 cancer types.

FIG. 5 is a result of confirming methylation information of a LIPE gene in a total of 33 cancer types.

FIG. 6 is a result of confirming methylation information of an INAFM1 gene in a total of 33 cancer types.

FIG. 7 is a result of confirming methylation information of an ATL1 gene in a total of 33 cancer types.

FIG. 8 is a result of confirming methylation information of a CELF6 gene in a total of 33 cancer types.

FIG. 9 is a result of confirming methylation information of an MTHFD2 gene in a total of 33 cancer types.

FIG. 10 is a result of confirming methylation information of a PAK1 gene in a total of 33 cancer types.

FIG. 11 is a result of confirming methylation information of an NXPE3 gene in a total of 33 cancer types.

FIG. 12 is a result of confirming methylation information of an SLC25A36 gene in a total of 33 cancer types.

FIG. 13 is a result of confirming methylation information of a VANGL2 gene in a total of 33 cancer types.

FIG. 14 is a result of confirming the accuracy of diagnosis for hepatocellular cancer of a total of 14 genes selected according to the present invention.

FIG. 15 is a result of confirming a difference in methylation between a tumor tissue (tumor) cell line group and a non-tumor tissue (others) cell line group.

FIGS. 16A and 16B are results of presenting the methylation levels of FAR1, PAK1, ATL1, and LIPE genes as $\Delta Ct + 10$ values after qMSP in liver cancer tissue and normal-adjacent tissue.

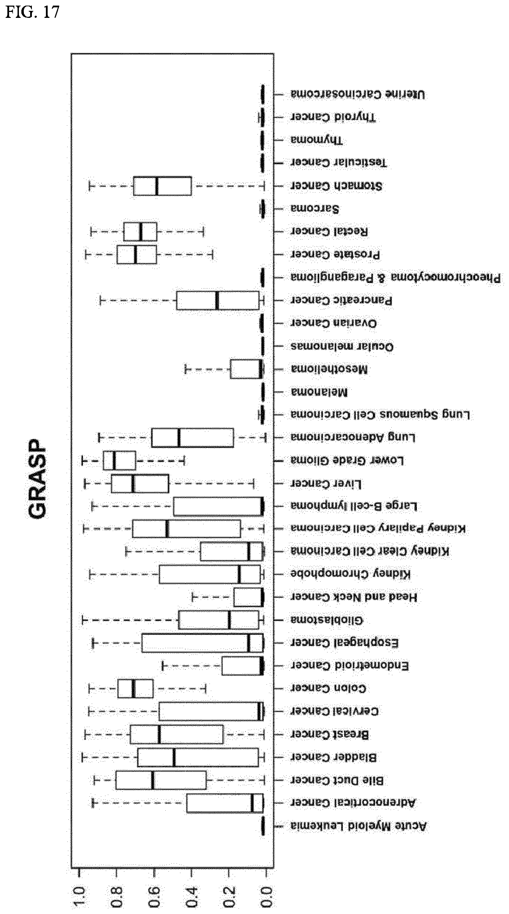

FIG. 17 is a result of confirming methylation information of a GRASP gene as

Comparative Example.

## MODES FOR THE INVENTION

Hereinafter, preferred Examples will be proposed in order to help in understanding of the present invention. However, the following Examples are just provided to more easily understand the present invention and the contents of the present invention are not limited by Examples.

**Example 1: Screening of hepatocellular cancer-specific methylated genes**

To screen methylated genes specifically found in hepatocellular cancer, a large-scale comparative study on methylation between cancer tissue obtained from cancer surgery of patients with colorectal cancer and normal tissue was conducted using data from three large-scale methylation microarray chips (see Table 2). The tumor tissue used in this study means cancer tissue of hepatocellular cancer, and the non-tumor tissue means tissues other than the cancer tissue including normal liver tissue.

[Table 2]

|  | dataset#1 | dataset#2 | dataset#3 | Total |
|---|---|---|---|---|
| Tumor | 66 | 37 | 377 | 480 |
| Non-tumor | 66 | 37 | 50 | 153 |

In order to screen hepatocellular cancer-specific methylated genes, DNA was extracted from each tissue, and the methylation levels of gene regions were confirmed using an Infinium Human Methylation 450 Beadchip microarray.

The DNA extracted from each tissue was converted through bisulfite treatment. Through this, a cytosine base was modified depending on the methylation of the DNA region. A probe used in the microarray experiment was specifically designed for methylation and unmethylation in order to confirm whether the cytosine base in a methylated region of a gene was modified.

The microarray experiment measured the methylation levels of the genes through about 450,000 (450 k) probes representing the methylated region of each gene, and a result of each probe derived from the experiment was presented as a beta value. The beta value had a value of 0 to 1, and it was determined that the closer to 1, the higher the methylation level of the corresponding gene region.

In order to identify differentially methylated regions (DMRs) between the tumor group and the non-tumor group, by using an empirical Bayes t-test, a Linear Models for Microarray Data (Limma) method, a gene region having a statistically significant methylation difference between the groups was identified.

The Limma method has been known to be least affected by an outlier among several methylation statistical analysis methods to identify the difference between the groups. Therefore, the method was a suitable method for finding cancer-specific markers because the method was less affected by abnormal measurement values of some samples. In the experiment, it was determined that there was a significant difference in methylation between the two groups as an adjusted p-value derived through the Limma method decreased.

In particular, in order to search for tumor-specific methylated regions, among gene regions with significant differences in beta values between tumor and non-tumor groups, gene regions with higher methylation in tumor tissue than in non-tumor tissue were selected as cancer-specific biomarker candidates.

As a result, as a result of limma analysis in each of the three datasets, compared with the non-tumor group, when comparing the tumor groups, gene regions having a significantly low p value and a large difference in beta value of 0.2 or more between the groups were selected as tumor-specific hypermethylated regions. As a result, among about 450,000 gene regions, 1,777 gene regions showing tumor-specific hypermethylation commonly in all the datasets were selected as biomarker candidates.

**Example 2: Screening of hepatocellular cancer-specific hypermethylated genes**

In the 1,777 gene regions as the biomarkers identified in Example 1, the methylation level of each corresponding region in tumors other than hepatocellular cancer was identified and compared to find a liver cancer-specific gene region among the biomarkers. As a result of analyzing the DNA methylation 450k array test results of The Cancer Genome Atlas (TCGA), a public cancer gene database, methylation information of gene regions corresponding to 33 cancer types was confirmed. Among them, as a result of identifying gene regions with significantly high beta values in liver cancer compared to hepatocellular cancer (called liver cancer) and other 32 cancer types, it was confirmed that in 42 gene regions among 1,777 gene regions, liver cancer-specific methylation occurred.

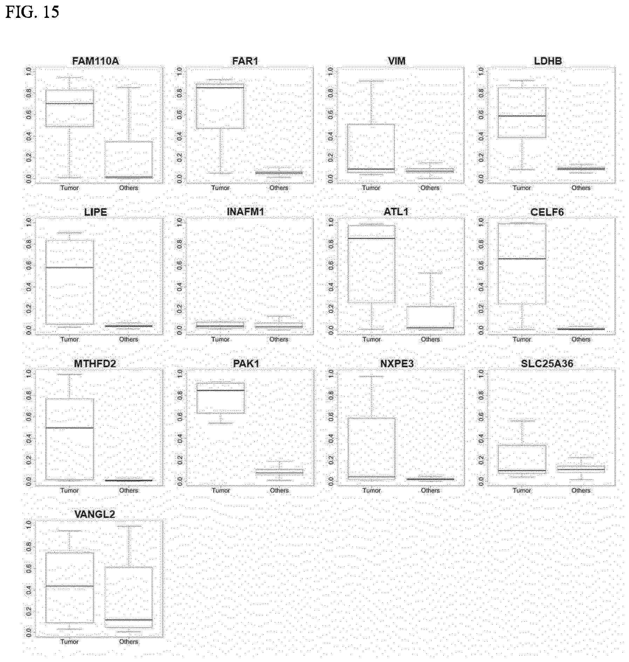

The methylation levels of the genes were as shown in FIG. 15 through the microarray experiment on tumor tissue (cancer tissue of hepatocellular cancer) and non-tumor tissue (tissue other than cancer tissue including normal liver tissue) for the genes. In the methylation level, the result of each probe derived through the experiment was represented as a beta value, and the beta value had 0 to 1, and it was determined that the closer to 1, the higher the methylation level of the corresponding gene region.

Meanwhile, in the case of a gene region where a difference in methylation was observed when comparing tumor tissue and non-tumor tissue of liver cancer, methylation may occur in cancers other than liver cancer. That is, liver cancer-specific methylation was not confirmed.

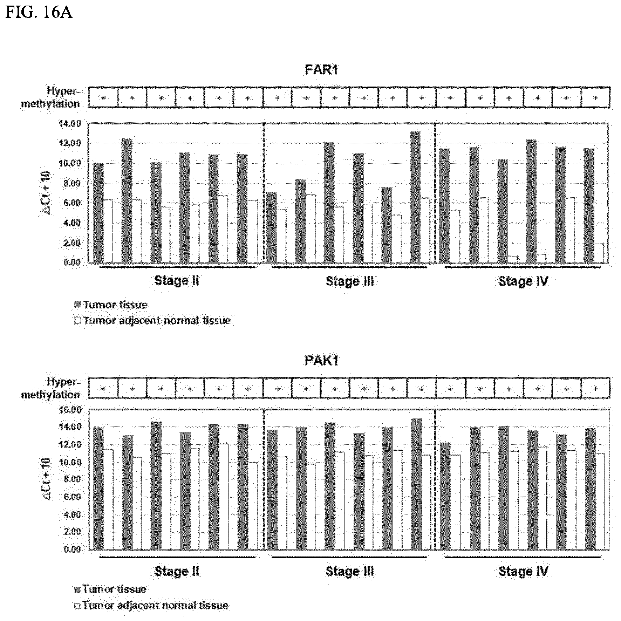

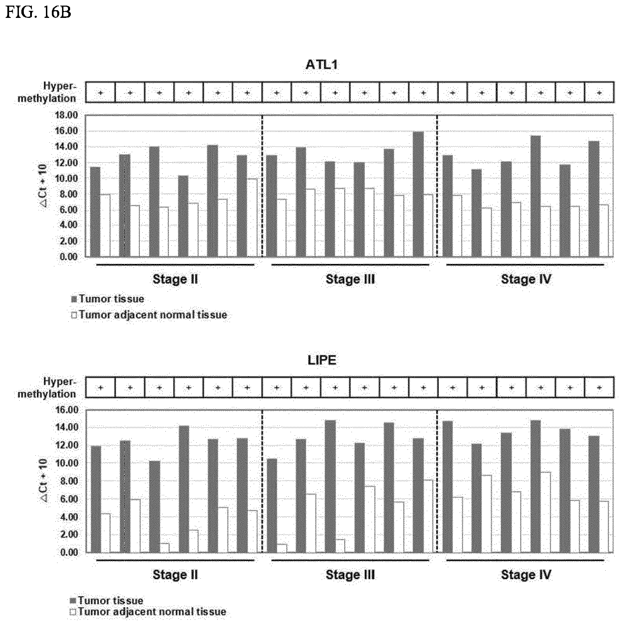

For example, a general receptor for phosphoinositides 1 associated scaffold protein (GRASP) gene was one of regions where the largest methylation difference between the tumor tissue and the non-tumor tissue was confirmed among the 1,777 gene regions identified in Example 1. However, as shown in FIG. 16, it was confirmed that high methylation occurred in liver cancer and at the same time, high methylation occurred even in various cancer types including prostate cancer, rectal cancer, stomach cancer, and the like.

The 33 cancer types were as follows: Acute Myeloid Leukemia, Adrenocortical cancer, Bile Duct cancer, Breast cancer, Cervical Cancer, Colon cancer, Endometrioid Cancer, Esophageal Cancer, Glioblastoma, Head and neck cancer, Kidney chromophobe, Kidney Clear cell carcinoma, Kidney papillary cell carcinoma, Large b-cell lymphoma, Liver cancer, Lower Grade Glioma, Lung adenocarcinoma, Melanoma, Mesothelioma, Ocular melanomas, Ovarian cancer, Pancreatic cancer, Pheochromocytoma & paraganglioma, Prostate cancer, Rectal cancer, Sarcoma, Stomach cancer, testicular cancer, Thymoma, Thyroid cancer, and Uterine carcinosarcoma.

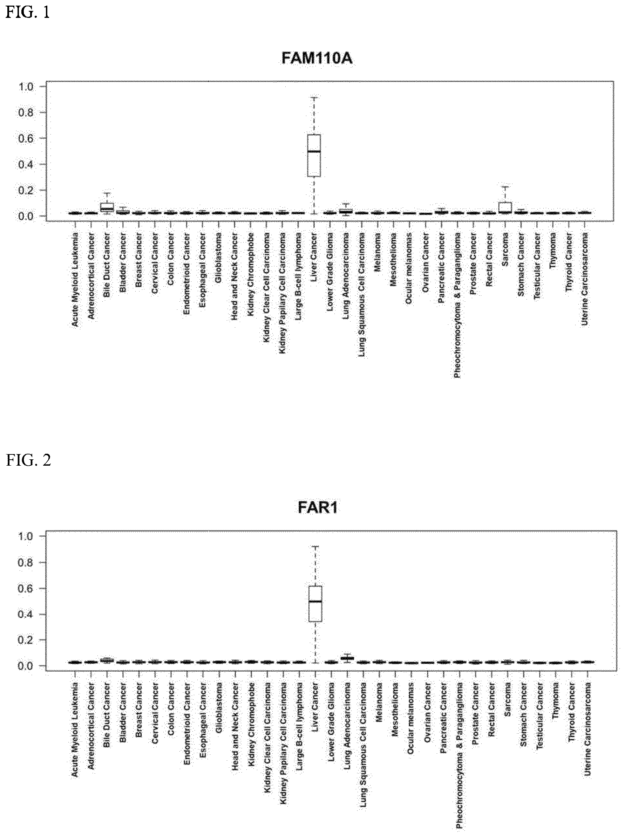

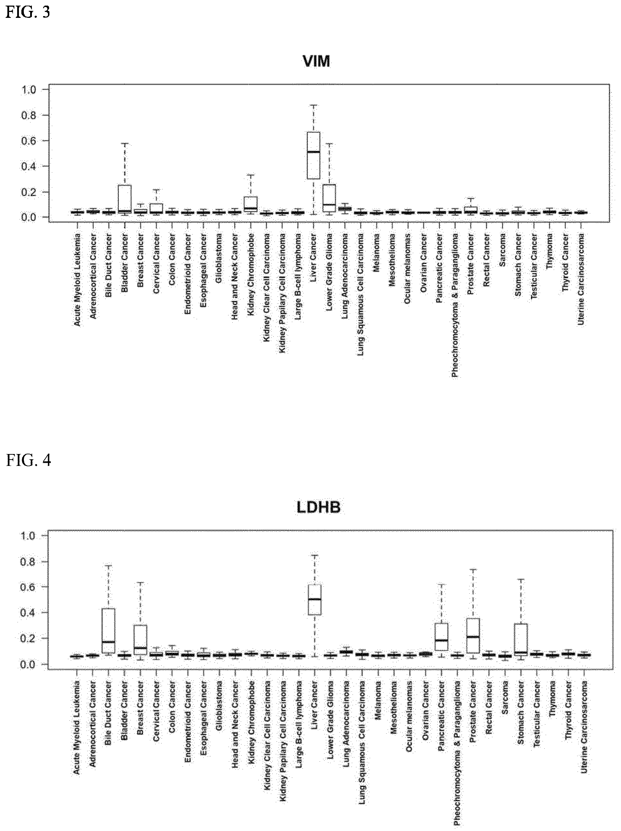

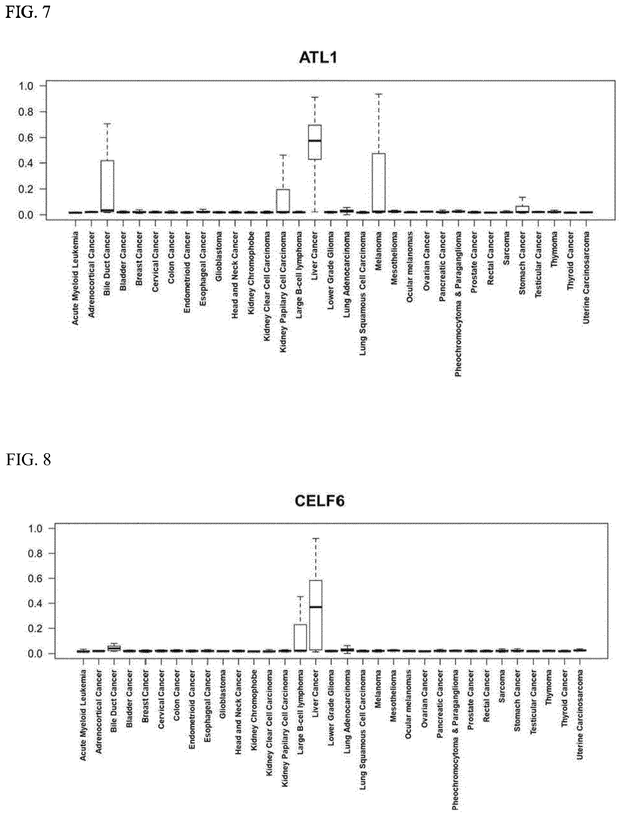

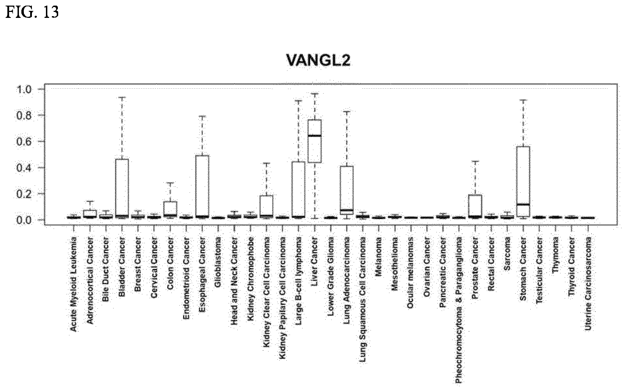

Among these gene regions, when the corresponding region was not a pseudogene, existed in a CpG island site, was located in a gene region selected between +/- 2000 bases (2 kb) from a transcription start site (TSS) of the gene, and existed in an autosome, the corresponding region was selected as a hepatocellular cancer-specific hypermethylation gene. As a result, as shown in Table 3 below, a total of 13 genes were selected (see FIGS. 1 to 13).

[Table 3]

| Symbol | Name | Location (Chromosome) | CpG Island |
|---|---|---|---|
| FAM110A | family with sequence similarity 110 member A | 20 | Island |
| FAR1 | fatty acyl-CoA reductase 1 | 11 | Island |
| VIM | Vimentin / VIM antisense RNA 1 | 10 | Island |

(continued)

| Symbol | Name | Location (Chromosome) | CpG Island |
|---|---|---|---|
| LDHB | lactate dehydrogenase B | 12 | Island |
| LIPE | Lipase E, hormone sensitive type / LIPE antisense RNA 1 | 19 | Island |
| INAFM1 | InaF motif containing 1 | 19 | Island |
| ATL1 | atlastin GTPase 1 | 14 | Island |
| CELF6 | CUGBP Elav-like family member 6 | 15 | Island |
| MTHFD2 | methylenetetrahydrofolate dehydrogenase (NADP+ dependent) 2, methenyltetrahydrofolate cyclohydrolase | 2 | Island |
| PAK1 | p21 (RAC1) activated kinase 1 | 11 | Island |
| NXPE3 | neurexophilin and PC-esterase domain family member 3 | 3 | Island |
| SLC25A36 | solute carrier family 25 member 36 | 3 | Island |
| VANGL2 | VANGL planar cell polarity protein 2 | 1 | Island |

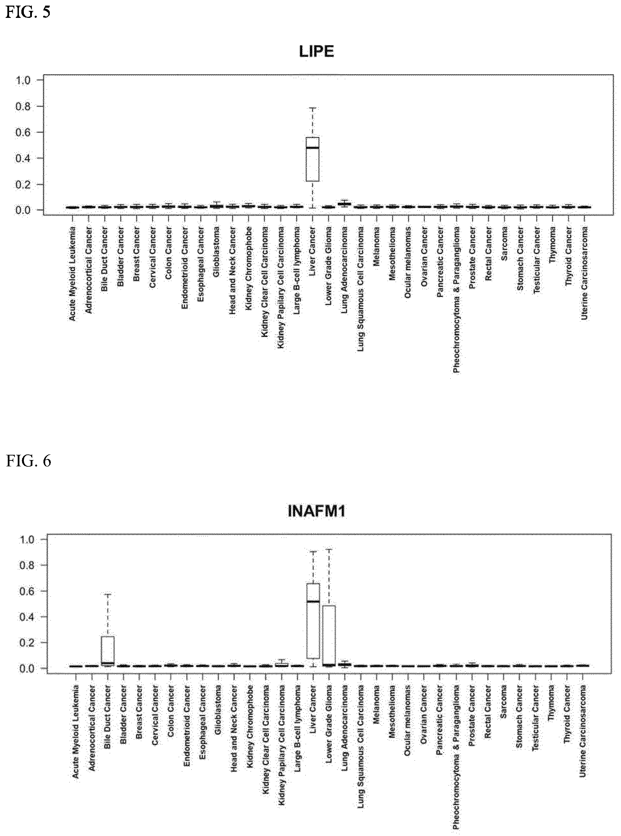

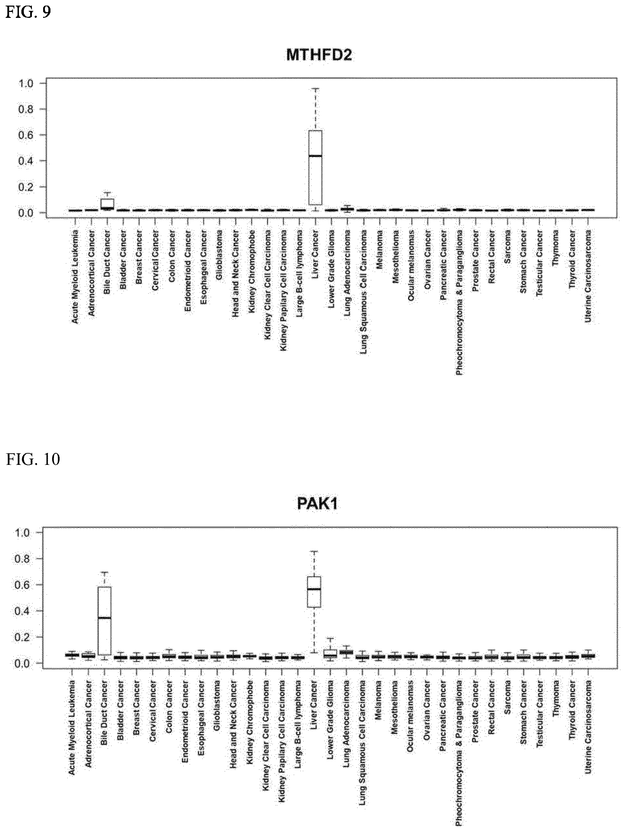

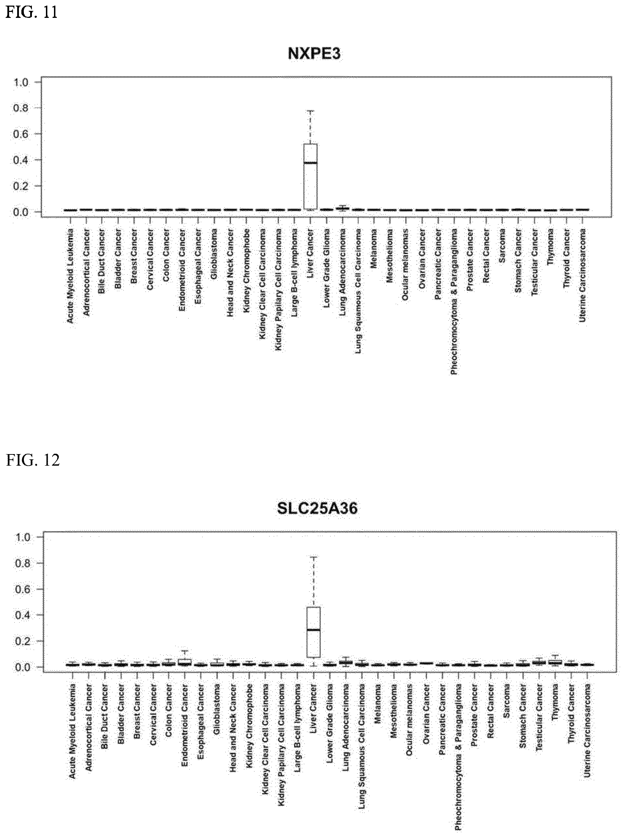

In particular, FAR1 (FIG. 2), LIPE (FIG. 5), MTHFD2 (FIG. 10), and NXPE3 (FIG. 12) showed distinct specific hypermethylation for liver cancer compared to other cancer types.

**Example 3: Confirmation of liver cancer specificity of selected genes in cell lines**

In order to confirm whether 13 selected genes exhibited liver cancer-specific methylation distinguished from other cancers, methylation patterns in 1,022 cancer cell lines derived from 14 tissues were analyzed using a public database. For the corresponding data, the Infinium Human Methylation 450 Beadchip microarray experiment was performed on DNA extracted from each cell line according to a manufacturer's standardized methylation analysis test procedure.

In the result values of the performed experiment, the methylation levels of genes were measured through about 450,000 probes as in Example 1, and the methylation value of each probe was presented as a beta value. The beta value had a value of 0 to 1, and it was determined that the closer to 1, the higher the methylation level of the corresponding gene region.

The 14 tissues were as follows: aerodigestive tract, blood, bone, breast, digestive system, kidney, lung, nervous system, pancreas, skin, soft tissue, thyroid, urogenital system, other tissue.

In order to confirm the liver cancer-specific methylation of the 14 selected genes, methylation data derived from 1,022 cell lines were largely classified into a liver cancer cell line group (n = 19) and a non-liver cancer cell line group (n = 1,003).

In order to identify differentially methylated regions (DMRs) between the two classified groups, by using an empirical Bayes t-test, a Linear Models for Microarray Data (Limma) method, gene regions having a statistically significant methylation difference between the groups were identified.

[Table 4]

| Difference in methylation between liver cancer cell line group and non-liver cancer cell line group of selected genes | | |
|---|---|---|
| Symbol | Difference (average $\Box\beta$) | adjusted p-value |
| FAM110A | 0.39 | 3.13e-06 |
| FAR1 | 0.59 | 1.94e-149 |
| VIM | 0.18 | 2.60e-06 |
| LDHB | 0.45 | 5.11e-27 |
| LIPE | 0.45 | 2.69e-46 |
| INAFM1 | 0.10 | 1.71e-01 |
| ATL1 | 0.44 | 1.26e-07 |

(continued)

| Difference in methylation between liver cancer cell line group and non-liver cancer cell line group of selected genes | | |
| --- | --- | --- |
| Symbol | Difference (average □β) | adjusted p-value |
| CELF6 | 0.51 | 1.87e-14 |
| MTHFD2 | 0.43 | 1.34e-121 |
| PAK1 | 0.55 | 3.49e-23 |
| NXPE3 | 0.30 | 3.87e-25 |
| SLC25A36 | 0.10 | 1.91e-15 |
| VANGL2 | 0.12 | 3.57e-01 |

In particular, in the case of FAR1, LIPE, MTHFD2, and NXPE3, which showed clear specific hypermethylation in liver cancer compared to other cancer types in actual patient samples, even in the case of analysis using cell lines, it was confirmed that as compared with other cancer cell lines, the liver cancer cell line had a remarkably low adjusted p-value to be specific to liver cancer. In addition, it was confirmed that genes such as LDHB and PAK1 showed distinct methylation in the liver cancer cell line compared to other cell lines.

**Example 4: Evaluation of diagnostic performance of liver cancer diagnostic marker candidates**

In order to confirm the usefulness of selected genes as diagnostic markers in hepatocellular cancer, the accuracy of diagnosis for hepatocellular cancer according to a methylation level was evaluated.

In order to evaluate the accuracy of diagnosis, sensitivity and specificity were used. A Receiver Operating Characteristic (ROC) curve that presented changes in sensitivity and specificity according to a cut-off value may be shown through the calculation of the sensitivity and specificity values for feasible cut-off values of consecutive diagnostic test measurement values. The accuracy of diagnosis may be measured by an area under the ROC curve (AUC). The AUC value has a value between 0.5 and 1, and it is evaluated that the higher the value, the higher the diagnosis accuracy. If the AUC value is 1, it is meant that the diagnosis result is a perfectly accurate test, but if the AUC value is 0.5, it is determined that the diagnosis result is the same as the random result.

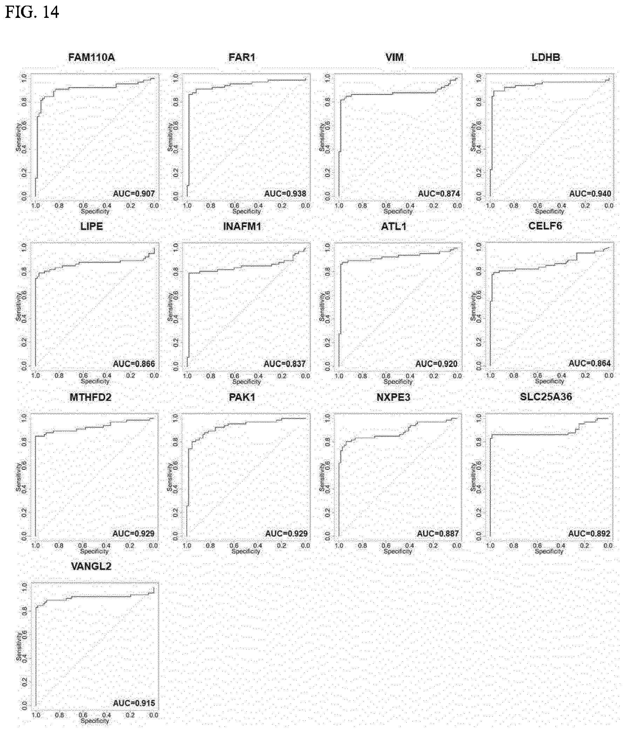

As a result of analyzing the cancer classification accuracy according to the methylation level between the non-tumor tissue and the tumor tissue using the selected genes by using a collected methylation dataset, as illustrated in FIG. 14, it was confirmed that all the selected genes had Area Under Curve (AUC) values of 0.860 or higher to have diagnosis accuracy, so that the selected genes were useful for diagnosing hepatocellular liver.

**Example 5: Measurement of liver cancer tissue qMSP-based methylation of selected genes**

Among 13 selected genes, an additional validation test using cancer tissue was performed with respect to 4 genes FAR1, PAK1, ATL1, and LIPE, which had a large difference □ methylation level between the non-tumor and tumor tissues and high average methylation levels in the tumor tissue. In order to confirm the liver cancer-specific methylation of the four selected genes in cancer tissue, a difference in methylation between cancer tissue and non-cancer tissue was measured using a quantitative methylation specific PCR (qMSP) technique. To this end, genomic DNA was isolated from a pair of cancer tissues and cancer-adjacent tissues of total 15 liver cancer patients, 5 for each stage from stage 2c to 4c, and treated with bisulfite, and then the amplification and methylation levels of specific gene regions were observed with respect to each of FAR1, PAK1, ATL1, and LIPE according to a generalized qMSP experimental method.

In addition, an ACTB gene, which was specifically bound to a gene region converted by bisulfite to be amplified and not related to methylation to standardize the amplified value of the corresponding region, was used as an internal control.

The methylation level of the bisulfite-converted DNA amplified by PCR was represented as $\Delta Ct + 10$, which was a value adjusted with a Cycle of Threshold (Ct) value of ACTB used as an internal control. $\Delta Ct + 10$ is defined as follows:

$$\Delta Ct + 10 = (Ct \text{ value of ACTB gene - Ct value of gene to be detected}) + 10$$

As shown in FIGS. 16A and 16B, it was confirmed that the methylation of each gene of FAR1, ATL1, PAK1, and LIPE showed a relatively high ΔCt + 10 value regardless of stage in colorectal cancer tissue compared to cancer-adjacent normal tissue, so that the four genes FAR1, ATL1, PAK1, and LIPE were hypermethylated in liver cancer. This is a result of actually showing that the methylation of the selected genes FAR1, ATL1, PAK1, and LIPE is effective as a biomarker for the diagnosis, particularly early diagnosis of liver cancer.

As such a result, it was found that the selected genes may be used even for the diagnosis of liver cancer.

## INDUSTRIAL APPLICABILITY

As described above, since hypermethylation of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 appears specifically in liver cancer, it is possible to accurately and rapidly liver cancer and early diagnose the liver cancer by using the composition, the kit, the chip or the method according to the present invention.

## Claims

1. A composition for diagnosing liver cancer comprising a preparation for measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.

2. The composition of claim 1, wherein the CpG site is located between +/- 2000 bases (2 kb) from a transcription start site of the gene.

3. The composition of claim 1, wherein the preparation for measuring the methylation level of the CpG site of the gene is selected from the group consisting of

   a compound of modifying an unmethylated cytosine or methylated cytosine base;
   primers specific to methylated sequences of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2; and
   primers specific to unmethylated sequences.

4. The composition of claim 3, wherein the compound of modifying the unmethylated cytosine base is bisulfite or a salt thereof and the compound of modifying the methylated cytosine base is a Tet protein.

5. A kit for diagnosing liver cancer comprising a primer pair for amplifying fragments including CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.

6. A nucleic acid chip for diagnosing liver cancer immobilized with probes capable of hybridizing with fragments including CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2.

7. A method for diagnosing liver cancer comprising steps of:

   measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 from samples of patients suspected of having liver cancer; and
   comparing the measured methylation levels with methylation levels of CpG sites of the same genes in a normal control sample.

8. The method of claim 7, wherein the method for measuring the methylation level is selected from the group consisting of a bisulfite-free detection method, methylation-specific polymerase chain reaction, real time methylation-specific polymerase chain reaction, PCR using a methylated DNA-specific binding protein, quantitative PCR, pyrosequencing, and bisulfite sequencing.

9. The method of claim 7, wherein the sample is selected from the group consisting of tissues, cells, blood, plasma, serum, feces, and urine.

10. Use of a preparation for measuring methylation levels of CpG sites of any one or more genes selected from the group consisting of FAM110A, FAR1, VIM, LDHB, LIPE, INAFM1, ATL1, CELF6, MTHFD2, PAK1, NXPE3, SLC25A36 and VANGL2 for preparing a preparation for diagnosing liver cancer.

FIG. 1

**FAM110A**

FIG. 2

**FAR1**

FIG. 3

**VIM**

FIG. 4

**LDHB**

FIG. 5

**LIPE**

FIG. 6

**INAFM1**

FIG. 7

**ATL1**

FIG. 8

**CELF6**

FIG. 9

MTHFD2

FIG. 10

PAK1

FIG. 11

NXPE3

FIG. 12

SLC25A36

FIG. 13

VANGL2

FIG. 14

FIG. 15

FIG. 16A

FIG. 16B

ATL1

LIPE

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20190127218
- WO 0126536 A
- US 20030148326 A1

### Non-patent literature cited in the description

- **SINGLETON et al.** DICTIONARY OF MICROBIOLOGY AND MOLECULAR BIOLOTY. 1994
- THE CAMBRIDGE DICTIONARY OF SCIENCE AND TECHNOLOGY. 1988
- **HALE ; MARHAM.** *THE HARPER COLLINS DICTIONARY OF BIOLOGY*
- *Nature Biotechnology,* 2019, vol. 37, 424-429
- **RONAGHI et al.** *Science,* 17 July 1998, vol. 281 (5375), 363-365
- **RONAGHI et al.** *Analytical Biochemistry,* 01 November 1996, vol. 242 (1), 84-9
- **RONAGHI et al.** *Analytical Biochemistry,* 15 November 2000, vol. 286 (2), 282-288
- **NYR, P.** *Methods Mol Biology,* 2007, vol. 373, 114
- **LIU, YIBIN et al.** *Nature Biotechnology,* 2019, vol. 37, 424-429
- **TAI et al.** *Plant Mol. Biol. Reporter,* 1990, vol. 8, 297-303
- **MURRAY et al.** *Nuc) Res.,* 1980, 4321-4325